# EUROPEAN PATENT SPECIFICATION

(11) **EP 1 894 585 B1**
(45) Date of publication and mention of the grant of the patent: **03.10.2012**
(21) Application number: 07253204.7
(22) Date of filing: 15.08.2007
(51) Int. Cl.: A61M 1/00

(54) **Adjustable aspiration device**
Einstellbare Aspirationsvorrichtung
Dispositif d'aspiration réglable

(30) Priority: 21.08.2006 US 507220
(43) Date of publication of application: 05.03.2008
(73) Proprietor: Tyco Healthcare Group LP, Mansfield, MA 02048 (US)
(72) Inventor: Callahan, Mark J., Medway Massachusetts 02053 (US); DeFazio, Perry, Enfield Connecticut (US); Guitarini, Mark, San Diego California 92101 (US)
(74) Representative: Gray, James

(56) References cited:
- WO-A-01/21995
- US-A- 3 537 457
- US-A- 4 417 874
- US-A- 4 867 747
- US-A- 5 078 603
- US-A- 5 217 465
- US-A1- 2002 065 482
- US-A1- 2003 105 422

## Description

### TECHNICAL FIELD

The present disclosure relates generally to surgical aspiration devices, and more particularly, relates to an aspiration device which is flexible and selectively extendable.

### BACKGROUND

Aspiration instruments, sometimes known as yankauers, are necessary surgical tools used to remove fluids, such as blood, from a patient's body during surgical procedures. Conventional yankauers incorporate a handle and a body that define a fluid passageway for removing fluid from the surgical site. The proximal end of the yankauer handle is connected to a vacuum source through a tube, hose, or the like. Yankauer handles may have a molded or ribbed gripping surface for improved handling and control of the yankauer.

Yankauer bodies are manufactured in a variety of configurations and sizes for a number of applications. Long bodied yankauers are used to aspirate in a remote body cavity, while narrow bodied, fine tipped yankauers are appropriate for more precise fluid removal. Aspirating fluid around sensitive organs and other body tissue is a delicate task. A yankauer with a relative short body provides increased control of the yankauer tip while aspirating fluids. A curved bodied yankauer may be necessary to aspirate fluid from a given cavity which is otherwise inaccessible or obstructed by other body organs.

During a surgical procedure, a yankauer may be required to aspirate fluids from a range of variously configured areas at different layers within the body cavity. Initially, a yankauer may be required to aspirate blood at the incision site. As the surgery progresses into the body cavity, fluid may collect in less convenient locations for aspirating. Maneuvering around delicate organs and other internal structures presents challenges to the operator. Any number of variously configured conventional yankauers may be necessary to effectively aspirate the body cavity during a surgical procedure. In the operating room, reducing the number of yankauers needed to perform a surgical procedure would reduce costs, in terms of number of devices needed, and save operating time. Therefore, it would be beneficial to have a yankauer body that is selectively adjustable to meet the various needs presented during a surgical procedure. Specifically, it would be beneficial for a yankauer to be capable of extension and retraction. It would further be beneficial for the yankauer to be able to be flexed or angulated.
US 2002/065482 is directed to a surgical suction instrument including a handle, a surgical cavity insertion tube, and a suction tube attachment fitting. The suction tube attachment fitting is in connection with the surgical cavity insertion tube at a location below a mechanical connection of the surgical cavity insertion tube and the handle. US 5078603 is directed to a filtering suction nozzle adapted for use in connection with a dental or medical suction system. The nozzle is suitable for use as a high speed suction tip and/or saliva ejector formed from an elongate tube defining a fluid flow path therethrough. US 4417874 is directed to a suction device having a plastic tube with a plurality of suction orifices. At least one portion of the tube is provided with ridges and intermediate grooves.
US5217465 discloses a flexible and steerable aspiration tip for use in microsurgery having a tip portion that can be remotely altered to access different areas of an operative site. The tip includes a flexible portion including a spring material that may be positioned in a straight or curved configuration. The aspiration tip may be combined with a handpiece assembly comprising the means to remotely alter configuration of the tip portion.

### SUMMARY

According to the present invention there is provided an aspiration instrument for removing fluid from a body cavity, which comprises an elongated member defining a longitudinal axis and having proximal and distal ends, the elongated member having a longitudinal conduit therethrough for passage of fluids and an opening for receiving fluids from the operative site, the opening being an axial opening provided in the distal end of the elongated member, the elongated member including a flexible and extendable portion adapted to be selectively adjusted to vary the length of and orientation relative to the longitudinal axis of the elongated member, the aspiration instrument including a handle, said handle being connected to the proximal end of the elongated member, wherein the handle has a proximal end configured to be operably connected to a vacuum source, the handle further defines a longitudinal lumen in fluid communication with the longitudinal conduit of the elongated member, and the distal end of the elongated member includes at least one vent extending through a wall portion of the elongated member and in fluid communication with the longitudinal conduit for releasing suction forces.
Preferably, the handle and elongated tubular body are monolithically formed. Alternatively, the elongated member is adapted for releasable attachment to the handle.

In one preferred embodiment, the flexible and extendable portion includes a general bellows configuration. The flexible and extendable portion may include a soft pliable material such as a soft metal or a soft polymer, and may be adapted to assume an arcuate configuration. The flexible and extendable portion may extend along at least a major portion of the length of the elongated member.

The elongated member may include at least one longitudinal rail adjacent the flexible and extendable portion. The at least one rail is adapted to increase stability of the flexible and extendable portion. At least one flange may be mounted adjacent one end of the flexible and extendable portion and adapted to receive the at least one rail. Preferably, a pair of flanges are mounted adjacent each end of the flexible and extendable portion and adapted to receive the at least one rail. The at least one rail is longitudinally fixed relative to one of the flanges and is adapted to move relative to the other flange to permit varying of the length and orientation of the flexible and extendable portion relative to the longitudinal axis of the elongated member.

A sleeve may be positionable about at least the flexible and extendable portion. The sleeve is adapted to releasably retain the flexible and extendable portion in an initial contracted position.

### BRIEF DESCRIPTION OF THE DRAWINGS

Preferred embodiments of the present disclosure will be better appreciated by reference to the drawings wherein:
FIG. 1 is a partial side cross-sectional view of an aspiration instrument constructed in accordance with the present disclosure including a flexible and extendable body portion in a retracted position;
FIG. 2 is a partial side cross-sectional view of the aspiration instrument of FIG. 1, illustrating the flexible and extendable body portion in an extended position;
FIG. 2A is a partial side cross-sectional view of the aspiration instrument of FIG. 1 illustrating the flexible and extendable body portion angulated;
FIG. 3 is partial side cross-sectional view of another embodiment of the aspiration instrument illustrating a flexible and extendable body portion in a retracted position;
FIG. 4 is partial side cross-sectional view of the aspiration instrument of FIG. 3 illustrating the flexible and extendable body portion in an extended position;
FIG. 5 is a partial side cross-sectional view of another embodiment of an aspirating instrument illustrating a flexible and extendable body portion in a retracted position;
FIG. 6 is a partial side cross-sectional side view of the aspiration instrument of FIG. 5 illustrating the flexible and extendable body portion in an extended position;
FIG. 7 is a side plan view of another embodiment of the aspiration instrument of the present disclosure;
FIG. 8 illustrates an aspiration kit including a handle and a plurality of variable length adjustable portions for selective attachment to the handle; and
FIG. 9 illustrates an alternate aspiration kit.

### DESCRIPTION OF THE PREFERRED EMBODIMENTS

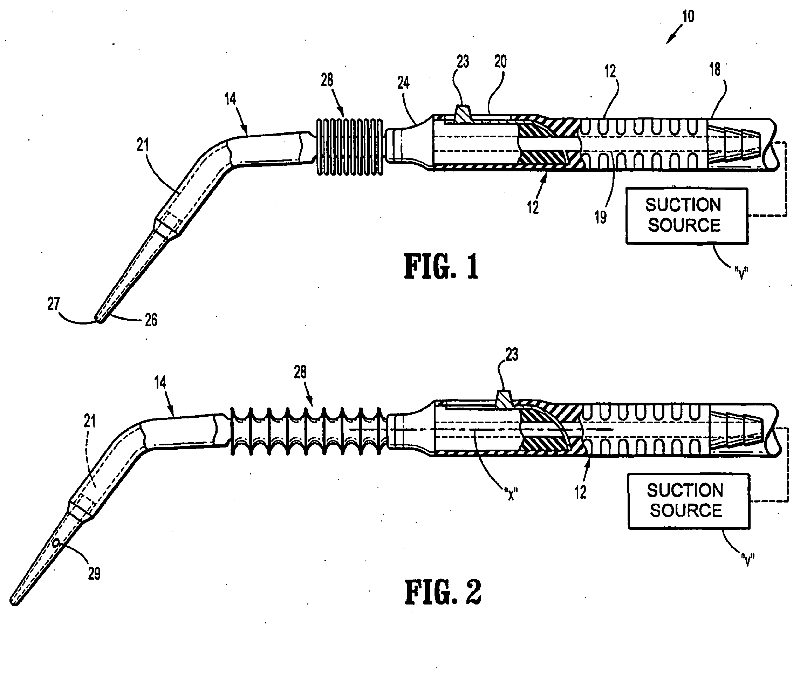

Referring now to the drawings wherein like reference numerals illustrate similar components throughout the several views, FIGS. 1-2A illustrate one preferred embodiment of the present disclosure. Yankauer 10 is contemplated for use in surgical procedures requiring the aspiration of fluids of differing viscosities and compositions from body cavities. Examples of such fluids of this type include blood, tissue fluid, tissue fragments, bone fragments, and rinsing fluids. Yankauer 10 consists of handle 12 and elongated body member 14 extending from handle 12. In one preferred embodiment, handle 12 and body member 14 are individual components; however, the handle 12 and the body member 14 may also be integrated to form a single unit and preferably are monolithically formed. Yankauer 10 may be formed of polymeric material and other plastics. Alternatively, yankauer 10 may be manufactured from a biocompatible metal, or metals, such as titanium or stainless steel. Yankauer 10 is operably connectable to a vacuum source "v" through the use of tubing, hosing, or other like material.

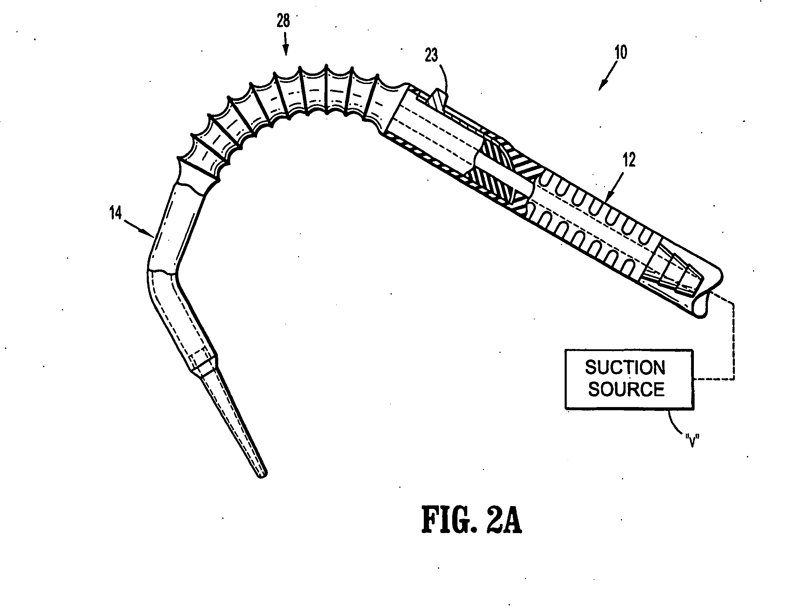

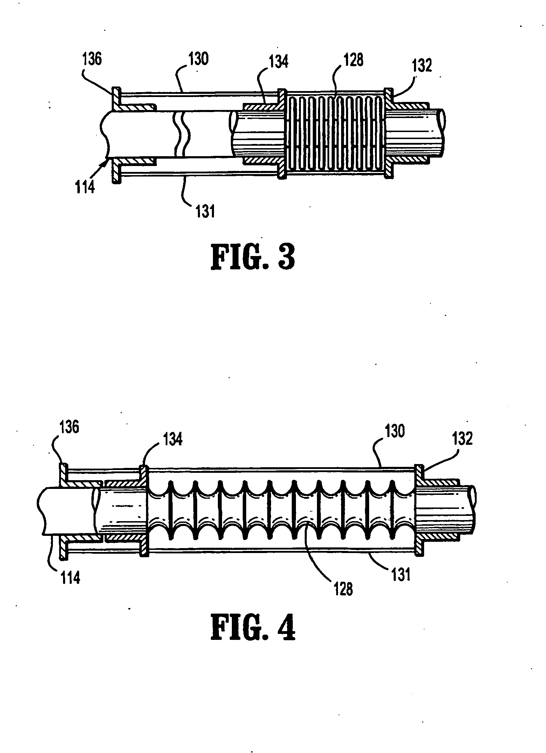

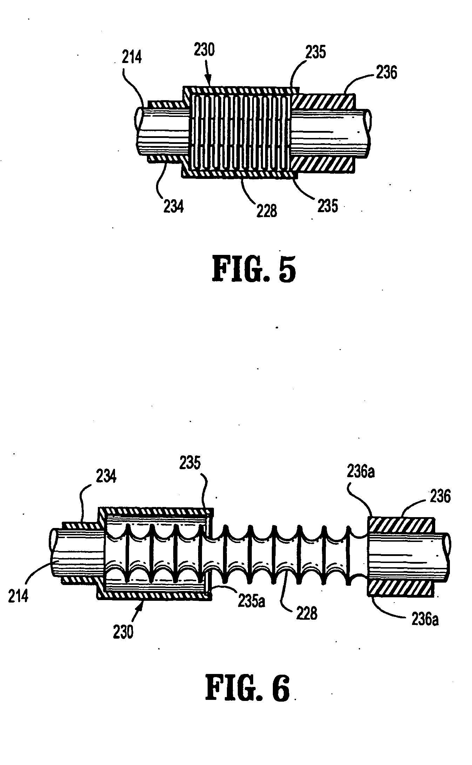

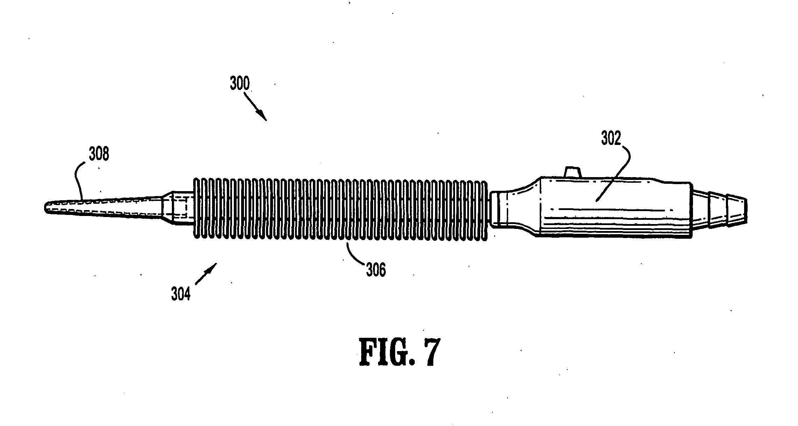

Handle 12 of yankauer 10 defines lumen or passageway 19 extending completely therethrough for the passage of fluids. Handle 12 defines proximal end 18, distal end 20 and middle portion 22, therebetween. Proximal end 18 of handle 12 is configured to be operably connected to a vacuum source "v". In the preferred embodiment, proximal end 18 includes a standard male connection or barbed port to frictionally receive a hose, tube or the like. Alternatively, proximal end 18 may incorporate a luer connector to connect the tubing to handle 12. Middle portion 22 of handle 12 defines an elongated tubular section. Middle portion 22 is configured for gripping and handling of yankauer 10, and may include serrations or ribs on its outer surface to facilitate engagement of the user. Middle portion 22 of handle 12 may be configured with longitudinal fins as disclosed in copending U.S. patent application 10/536,545, incorporated herein by reference. Middle portion 22 of handle 12 is configured with a valve member 23 for controlling the suction delivered to distal end 20 of yankauer 10. Valve member 23 may be of any number of designs. As shown, valve member 23 includes a slidable lever that operates to selectively obstruct passageway 19 through handle 12. FIGS. 1 and 2 illustrate valve member 23 in the open and closed positions, respectively. Distal end 20 of handle 12 is configured to receive proximal end 24 of body member 14. The subject matter of the present disclosure can be adapted to work with yankauer handles of all sizes and configurations.
Elongated body member 14 defines passageway 21 therethough in fluid communication with passageway 19 of handle 12 for the removal of fluids from the body cavity. Body member 14 has proximal end 24 and distal end 26. Distal end 26 of body member 14 defines an axial opening 27 for receiving fluids from the operative site. Proximal end 24 of body member 14 is configured to be operably connected with distal end 20 of handle 12. Mechanical fasteners may be employed to fluidly connect body member 14 and handle 12. In one preferred embodiment, proximal end 24 is frictionally received by distal end 20 of handle 12, and is secured in place with an adhesive or mechanical fastener. In the alternative, as discussed hereinabove, handle 12 and body member 14 may be a single monolithically formed unit. Distal end 26 of body member 14 may include one or more apertures 29 for venting the suction received from vacuum source "v". All known connection and body member configurations, with and without vents, have been contemplated by this disclosure and are adaptable for use with the disclosed subject matter.
Body member 14 further defines selectively adjustable portion 28 defining a bellows or an accordion-like configuration. The accordion-like configuration permits adjustable portion 28 of body member 14 to be selectively extendable. The accordion-like configuration also allows body member 14 to be selectively flexed or angulated relative to longitudinal axis "x" to adapt the shape of the yankauer to the body cavity being aspirated. Adjustable portion 28 may be used in a fully extended position of FIG. 2, a fully compressed or retracted position of FIG. 1 or any contemplated intermediate position. Adjustable portion 28 may be extended or retracted and angulated, as shown in FIG. 2A, during a surgical procedure, and may assume an arcuate configuration. Adjustable portion 28 may be formed of a flexible polymeric material or a soft metal. Reinforcement material may be integrated into adjustable portion 28. Such reinforcement material includes materials to increase the rigidity of adjustable portion 28 whereby the adjustable portion 28 may retain any angulated or extended orientation contemplated. In this regard, the surgeon may angulate and/or extend adjustable portion 28 to a desired orientation and then introduce the adjustable portion 28 within the patient's body. Manufacturing techniques to achieve the objective are readily appreciated by one skilled in the art.
The preferred embodiment of body member 14 is manufactured using blow molding, however, all other processes of manufacture have been contemplated by this disclosure. Body member 14 may be constructed of plastic, or other like. Body member 14 may comprise soft metallic material or soft polymeric material. Body member 14 may also be disposable.
Referring now to FIGS. 3-4, in an alternate embodiment, body member 114 includes a rail structure to provide additional support to adjustable portion 128. The rail structure includes flange 132 which is secured to body member 114 adjacent the proximal side of adjustable portion 128 and flange 134 secured to body member 114 adjacent the distal side of the adjustable portion 128. A pair of longitudinal rails 130, 131 extends through openings of flanges 132, 134. A third flange 136 is mounted to body member 114 distal of adjustable portion 128. During extension of adjustable portion 128 shown in FIG. 4, flange 134 traverses rails 130, 131 to permit the adjustable portion 128 to selectively extend its length. In instances where middle portion 128 is fully or partially extended and the yankauer tip comes into contact with a structure in the body cavity, rails 130, 131 will increase the stability of middle portion 128. Rails 130, 131 may be flexed or bent to accommodate the configuration necessary to support middle portion 128.
Referring now to FIGS. 5 and 6, in another embodiment, body portion 214 includes a sleeve 230 for selectively securing adjustable portion 228 in the retracted position. Sleeve 230 is an annular ring constructed from plastic, metal or the like. Sleeve 230 is securely affixed at one end to elongated body portion 214 distal of adjustable portion 128 through flange 234. The other end or proximal end 235 of sleeve 230 is adapted to be mounted about receiving mount 236. Receiving mount 236 is secured to body portion 214 adjacent the proximal side of adjustable portion 228. Receiving mount 236 may be configured with a lip 236a for frictionally engaging sleeve 230 as shown in FIG. 6. It is envisioned that proximal end 235 of sleeve 230 may be releasably connected to receiving mount 236 with any known means, including mechanical fasteners.
In a retracted position, adjustable portion 228 is completely contained within sleeve 230 (FIG. 5). Sleeve 230 is frictionally received about lip 236a of receiving mount 236. To extend adjustable portion, the distal force is applied to sleeve 230 to overcome its locking, frictional relationship with receiving mount 236. Sleeve 230 provides added structure to adjustable portion 228 of the yankauer when in the retracted position. The added stability provided by sleeve 230 allows the yankauer to be handled with greater control and accuracy when aspirating fluids from within the body cavity and a relatively short yankauer body is required.
In an extended position, adjustable portion 228 is only partially contained within sleeve 230. Sleeve may include a seal 235a for preventing fluid from entering sleeve 230. Any type of seal is envisioned including septum seals, duckbill seals, slit seals, etc. Alternate embodiments of sleeve 230 are contemplated by this disclosure, including a partially encasing sleeve, as well as a flexible sleeve.
Referring now to FIG. 7, another embodiment of the present disclosure is illustrated. In accordance with this embodiment, aspiration instrument 300 includes handle 302 and elongated member 304 extending from the handle 302. Handle 302 is preferably rigid for engagement by the clinician. Elongated member 304 has adjustable portion 306 and aspiration tip 308 at the distal end of the elongated member 304. Adjustable portion 306 may extend along a major portion of the length (e.g., greater than 50%) of elongated member 304 to provide greater flexibility to the elongated member 304. Such feature may enable the clinician to orient adjustable portion in a variety of configurations (e.g., in a serpentine arrangement) to follow, e.g., a serpentine path through the tissue. Adjustable portion 306 may be extensible and/or retractable in a manner similar to the prior embodiments.

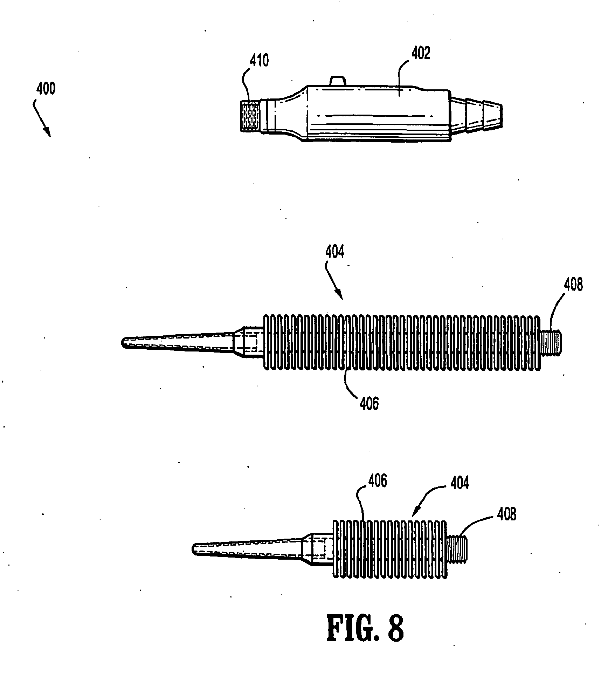

FIG. 8 illustrates an aspiration kit 400 which includes handle 402 and a plurality of elongated members 404 which are releasably connectable to the handle 402. Each elongated member 404 includes adjustable portion 406 which vary in their respective lengths. In this regard, the clinician may choose an elongated member 404 with a desired length adjustable portion 406 depending on the location of the aspiration site and connect the respective elongated member 404 to handle 402. Means for releasably mounting each elongated member 404 to handle 402 are readily envisioned by one skilled in the art. A threaded coupling may be utilized where elongated member 404 includes proximal external thread 408 and handle 402 incorporates a cooperating internal thread 410. The reverse arrangement of threading is also envisioned. Interference fits, bayonet couplings, snap fit mechanisms may also be utilized.

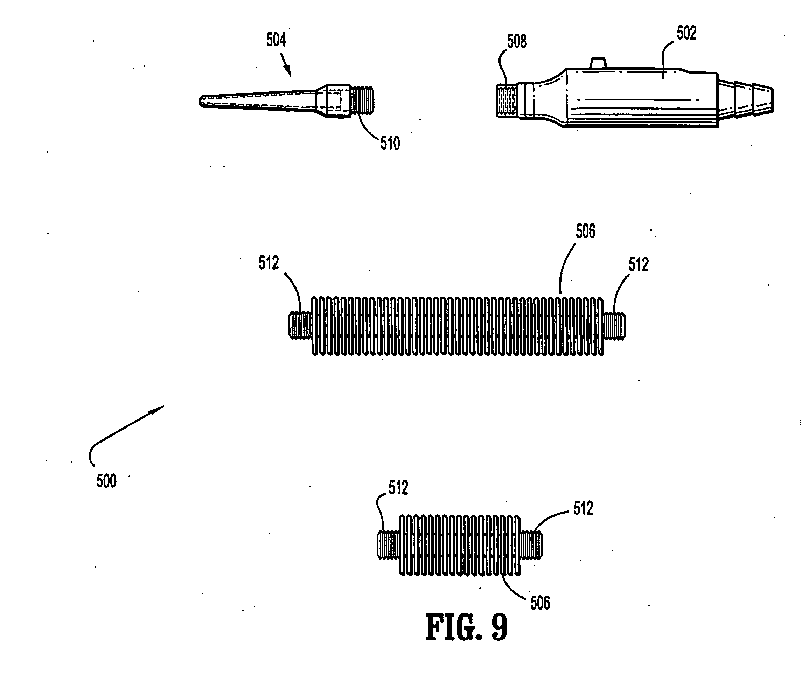

FIG. 9 illustrates an alternate aspiration kit 500 including handle 502, aspiration tip 504 and a plurality of variable length adjustable portions 506. Each adjustable portion 506 is releasably mountable to both handle 502 and aspiration tip 504 via a mechanical connection (e.g., threaded coupling, bayonet coupling, interference fit, etc.) Threads are shown schematically as reference numerals 508, 510, 512 on handle 502, aspiration tip 504 and adjustable portions 506, respectively. In this arrangement, handle 502 and aspiration kit 504 are standard whereby the clinician may select the desired length adjustable portion 506 depending on the location of the aspiration site.

It will be understood that various modifications may be made to the embodiments disclosed herein. Therefore, the above description should not be construed as limiting, but merely as exemplifications of preferred embodiments. Those skilled in the art will envision other modifications within the scope of the claims append hereto.

## Claims

1. An aspiration instrument (10,300,400) for removing fluid from a body cavity, which comprises an elongated member (14,114,214,304,404) defining a longitudinal axis (x) and having proximal and distal ends (24,26), the elongated member (14,114,214,304,404) having a longitudinal conduit (21) therethrough for passage of fluids and an opening for receiving fluids from the operative site, the opening (27) being an axial opening provided in the distal end (26) of the elongated member (14,114,214,304,404), the elongated member (14,114,214,304,404) including a flexible and extendable portion (28,128,228,306,406) adapted to be selectively adjusted to vary the length of and orientation relative to the longitudinal axis (x) of the elongated member (14,114,214,304,404), the aspiration instrument including a handle (12,302,402), said handle (12,302,402) being connected to the proximal end (24) of the elongated member (14,114,214,304,404), wherein the handle (12,304,402) has a proximal end (18) configured to be operably connected to a vacuum source, the handle (12,304,402) further defines a longitudinal lumen (19) in fluid communication with the longitudinal conduit (21) of the elongated member (14,114,214,304,404) **characterized in that** the distal end (26) of the elongated member (14,114,214,304,404) includes at least one vent (29) extending through a wall portion of the elongated member and in fluid communication with the longitudinal conduit (21) for releasing suction forces.

2. The aspiration instrument (10,300,400) of claim 1 wherein the handle (12,302,402) and elongated tubular body (14,114,214,304,404) are monolithically formed.

3. The aspiration instrument (10,300,400) according to claim 1 wherein the flexible and extendable portion (28,128,228,306,406) includes a general bellows configuration.

4. The aspiration instrument (10,300,400) according to claim 1 wherein the flexible and extendable portion (28,128,228,306,406) comprises a soft pliable material.

5. The aspiration instrument (10,300,400) according to claim 4 wherein the soft pliable material of the flexible and extendable portion (28,128,228,306,406)comprises a soft metal.

6. The aspiration instrument (10,300,400) according to claim 4 wherein the soft pliable material includes a soft polymer.

7. The aspiration instrument (10,300,400) according to claim 1 the flexible and extendable portion (28,128,228,306,406) is adapted to assume an arcuate configuration.

8. The aspiration instrument (10,300,400) according to claim 1 including a sleeve (230) positionable about at least the flexible and extendable portion (228), and adapted to releasably retain the flexible and extendable portion (228) in an initial contracted position.

9. The aspiration instrument (10,300,400) according to claim 1 wherein the flexible and extendable portion (28,128,228,306,406) extends along at least a major portion of the length of the elongated member (14,114,214,304,404).

10. The aspiration instrument (10,300,400) according to claim 1 wherein the elongated member (14,114,214,304,404) is adapted for releasable attachment to the handle (12,302,402).

## Patentansprüche

1. Aspirationsinstrument (10, 300, 400) zum Entfernen von Fluid aus einem Körperhohlraum, das ein langgestrecktes Element (14, 114, 214, 304, 404) umfasst, das eine Längsachse (x) definiert und ein proximales und ein distales Ende (24, 26) besitzt, wobei das langgestreckte Element (14, 114, 214, 304, 404) eine durch es verlaufende longitudinale Leitung (21) für den Durchgang von Fluiden und eine Öffnung für die Aufnahme von Fluiden von dem Operationsort besitzt, wobei die Öffnung (27) eine axiale Öffnung ist, die in dem distalen Ende (26) des langgestreckten Elements (14, 114, 214, 304, 404) vorgesehen ist, wobei das langgestreckte Element (14, 114, 214, 304, 404) einen biegsamen und ausfahrbaren Abschnitt (28, 128, 228, 306, 406) enthält, der dafür ausgelegt ist, wahlweise eingestellt zu werden, um die Länge und die Orientierung in Bezug auf die Längsachse (x) des langgestreckten Elements (14, 114, 214, 304, 404) zu variieren, wobei das Aspirationsinstrument einen Griff (12, 302, 402) umfasst, wobei der Griff (12, 302, 402) mit dem proximalen Ende (24) des langgestreckten Elements (14, 114, 214, 304, 404) verbunden ist, wobei der Griff (12, 302, 402) ein proximales Ende (18) besitzt, das konfiguriert ist, mit einer Unterdruckquelle betriebstechnisch verbunden zu werden, wobei der Griff (12, 302, 402) ferner einen longitudinalen Hohlraum (19) in Fluidkommunikation mit der longitudinalen Leitung (21) des langgestreckten Elements (14, 114, 214, 304, 404) definiert, **dadurch gekennzeichnet, dass** das distale Ende (26) des langgestreckten Elements (14, 114, 214, 304, 404) wenigstens einen Fluiddurchlass (29) aufweist, der durch einen Wandabschnitt des langgestreckten Elements verläuft und mit der longitudinalen Leitung (21) in Fluidkommunikation steht, um Saugkräfte zu entlasten.

2. Aspirationsinstrument (10, 300, 400) nach Anspruch 1, wobei der Griff (12, 302, 402) und der langgestreckte, rohrförmige Körper (14, 114, 214, 304, 404) monolithisch ausgebildet sind.

3. Aspirationsinstrument (10, 300, 400) nach Anspruch 1, wobei der biegsame und ausfahrbare Abschnitt (28, 128, 228, 306, 406) eine allgemeine Balgkonfiguration besitzt.

4. Aspirationsinstrument (10, 300, 400) nach Anspruch 1, wobei der biegsame und ausfahrbare Abschnitt (28, 128, 228, 306, 406) ein weiches, geschmeidiges Material enthält.

5. Aspirationsinstrument (10, 300, 400) nach Anspruch 4, wobei das weiche, geschmeidige Material des biegsamen und ausfahrbaren Abschnitts (28, 128, 228, 306, 406) ein Weichmetall enthält.

6. Aspirationsinstrument (10, 300, 400) nach Anspruch 4, wobei das weiche, geschmeidige Material ein weiches Polymer enthält.

7. Aspirationsinstrument (10, 300, 400) nach Anspruch 1, wobei der biegsame und ausfahrbare Abschnitt (28, 128, 228, 306, 406) dafür ausgelegt ist, eine gekrümmte Konfiguration anzunehmen.

8. Aspirationsinstrument (10, 300, 400) nach Anspruch 1, das eine Hülse (230) aufweist, die zumindest um den biegsamen und ausfahrbaren Abschnitt (228) positionierbar ist und dafür ausgelegt ist, den biegsamen und ausfahrbaren Abschnitt (228) in einer anfänglichen kontrahierten Position lösbar zu halten.

9. Aspirationsinstrument (10, 300, 400) nach Anspruch 1, wobei der biegsame und ausfahrbare Abschnitt (28, 128, 228, 306, 406) wenigstens längs eines wesentlichen Abschnitts der Länge des langgestreckten Elements (14, 114, 214, 304, 404) verläuft.

10. Aspirationsinstrument (10, 300, 400) nach Anspruch 1, wobei das langgestreckte Element (14, 114, 214, 304, 404) dafür ausgelegt ist, am Griff (12, 302, 402) lösbar befestigt zu werden.

## Revendications

1. Instrument d'aspiration (10, 300, 400) pour extraire un fluide d'une cavité du corps, qui comprend un élément allongé (14, 114, 214, 304, 404) définissant un axe longitudinal (x) et ayant des extrémités proximale et distale (24, 26), l'élément allongé (14, 114, 214, 304, 404) comportant un conduit longitudinal (21) le traversant pour le passage des fluides et une ouverture destinée à recevoir les fluides depuis le champ opératoire, l'ouverture (27) étant une ouverture axiale ménagée dans l'extrémité distale (26) de l'élément allongé (14, 114, 214, 304, 404), l'élément allongé (14, 114, 214, 304, 404) comprenant une partie flexible et extensible (28, 128, 228, 306, 406) apte à être ajustée sélectivement pour faire varier la longueur, et l'orientation par rapport à l'axe longitudinal (x), de l'élément allongé (14, 114, 214, 304, 404), l'instrument d'aspiration comprenant un manche (12, 302, 402), ledit manche (12, 302, 402) étant relié à l'extrémité proximale (24) de l'élément allongé (14, 114, 214, 304, 404), le manche (12, 302, 402) ayant une extrémité proximale (18) conçue pour être fonctionnellement reliée à une source de vide, le manche (12, 302, 402) définissant, en outre, une lumière longitudinale (19) en communication fluidique avec le conduit longitudinal (21) de l'élément allongé (14, 114, 214, 304, 404), **caractérisé en ce que** l'extrémité distale (26) de l'élément allongé (14, 114, 214, 304, 404) comprend au moins un orifice d'aération (29) traversant une partie de paroi de l'élément allongé et en communication fluidique avec le conduit longitudinal (21) pour supprimer les forces d'aspiration.

2. Instrument d'aspiration (10, 300, 400) selon la revendication 1, dans lequel le manche (12, 302, 402) et le corps tubulaire allongé (14, 114, 214, 304, 404) sont formés d'un seul bloc.

3. Instrument d'aspiration (10, 300, 400) selon la revendication 1, dans lequel la partie flexible et extensible (28, 128, 228, 306, 406) a une configuration générale de soufflet.

4. Instrument d'aspiration (10, 300, 400) selon la revendication 1, dans lequel la partie flexible et extensible (28, 128, 228, 306, 406) comprend un matériau souple et conformable.

5. Instrument d'aspiration (10, 300, 400) selon la revendication 4, dans lequel le matériau souple et conformable de la partie flexible et extensible (28, 128, 228, 306, 406) comprend un métal tendre.

6. Instrument d'aspiration (10, 300, 400) selon la revendication 4, dans lequel le matériau souple et conformable comprend un polymère tendre.

7. Instrument d'aspiration (10, 300, 400) selon la revendication 1, dans lequel la partie flexible et extensible (28, 128, 228, 306, 406) est apte à adopter une configuration arquée.

8. Instrument d'aspiration (10, 300, 400) selon la revendication 1, comprenant un manchon (230) positionnable autour d'au moins la partie flexible et extensible (228), et apte à retenir, de manière amovible, la partie flexible et extensible (228) dans une position contractée initiale.

9. Instrument d'aspiration (10, 300, 400) selon la revendication 1, dans lequel la partie flexible et extensible (28, 128, 228, 306, 406) s'étend le long d'au moins une partie principale de la longueur de l'élément allongé (14, 114, 214, 304, 404).

10. Instrument d'aspiration (10, 300, 400) selon la revendication 1, dans lequel l'élément allongé (14, 114, 214, 304, 404) est apte à être fixé de manière amovible au manche (12, 302, 402).
